# EUROPÄISCHE PATENTANMELDUNG

(11) **EP 1 595 878 A1**
(43) Veröffentlichungstag der Anmeldung: **16.11.2005**
(21) Anmeldenummer: 04011598.2
(22) Anmeldetag: 15.05.2004
(51) Int. Cl.: C07D 401/12, C07D 403/12, C07D 471/04, A61K 31/404, A61P 35/00

(54) **Indolderivate mit Apoptose induzierender Wirkung**

(71) Anmelder: Zentaris GmbH, 60314 Frankfurt/Main (DE)
(72) Erfinder: Gerlach, Matthias, Dr., 63636 Brachttal (DE); Schuster, Tilmann, Dr., 60439 Frankfurt (DE); Emig, Peter, Dr., 63486 Bruchköbel (DE); Schmidt, Peter, 61137 Schöneck (DE); Baasner, Silke, Dr., 61137 Schöneck (DE); Eckhard, Günther, Dr., 63477 Maintal (DE)

(57) **Zusammenfassung**

Gegenstand der Erfindung sind Indol-Derivate, die als Arzneimittel zur Behandlung von Tumorerkrankungen, insbesondere bei Arzneimittelresistenz gegen andere Wirkstoffe und bei metastasierendem Karcinom eingesetzt werden.

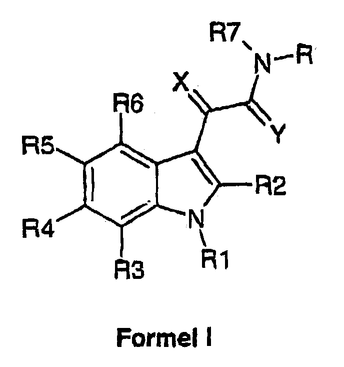

## Beschreibung

Die vorliegende Erfindung betrifft neue Indol-Derivate mit verbesserter biologischer Wirkung, verbesserter Verträglichkeit und verbesserter oraler Bioverfügbarkeit, die als Arzneimittel zur Behandlung von Tumorerkrankungen, insbesondere bei Arzneimittelresistenz gegen andere Wirkstoffe und bei metastasierendem Karcinom eingesetzt werden.

Die Behandlung von Krebskrankheiten hat in der Medizin eine große Bedeutung. Es besteht ein weltweiter Bedarf an gut wirksamen Krebstherapien für eine patientengerechte und zielorientierte Behandlung. Dies zeigt sich in der großen Anzahl von wissenschaftlichen Arbeiten, die auf dem Gebiet der angewandten Onkologie bzw. der Grundlagenforschung zur Krebstherapie in letzter Zeit erschienen sind.

Die Wirkung der Tumorhemmstoffe beruht hierbei auf den unterschiedlichsten Mechanismen und ist nur zum Teil bekannt. Es ist nicht ungewöhnlich, dass für bekannte Tumormedikamente neue Wirkmechanismen gefunden werden. Auch für die erfindungsgemäßen Verbindungen ist dies zu erwarten. Viele Tumormedikamente wirken über Mechanismen wie die Blockade des Zellteilungsmechanismus der Zelle, die Unterbindung der Versorgung des Tumors mit Nährstoffen und Sauerstoff (Antiangiogenese), die Verhinderung der Metastasierung, die Verhinderung des Empfangs und der Weiterleitung von Wachstumssignalen an die Tumorzelle oder die Abdrängung der Tumorzelle in den programmierten Zelltod (Apoptose).

Aufgrund unterschiedlicher Wirkmechanismen u.a. der Wechselwirkung mit unterschiedlichen intrazellulären Targets werden die klinisch relevanten Cytostatika häufig in Kombination gegeben, um synergistische Therapieeffekt zu erzielen.

Indolderivate finden als pharmakodynamisch aktive Verbindungen und als Synthesebausteine in der pharmazeutischen Chemie eine vielfältige Verwendung.

In den Dokumenten WO99/51224 A1 und WO01/22954 A1 werden Indol-3-yl-Derivate mit Antitumorwirkung, die mit zahlreichen Gruppen u.a. auch mit 2-, 3-, 4- und 8-Chinolin- oder 2-,3-,4-,5- und 6-Pyridin-Resten substituiert sein können, beschrieben. In Beispiel 60 wird eine 2-Methyl-8-Chinolinyl-Gruppe als Substituent an der Amidgruppe genannt. Es werden jedoch keine biologischen Eigenschaften genannt.

In der WO99/55696 A1 werden substituierte Hydroxyindole als Phosphodiesterase 4-Inhibitoren beschrieben. Eine antitumorale Aktivität für die erfindungsgemäßen Verbindungen ist weder beschrieben noch nahegelegt.

In der WO 02/08225 A1 werden 2-(1H-Indol-3yl)-2-oxoacetamidderivate mit Antitumorwirkung gegenüber soliden Tumoren beschrieben. Konkrete Ausführungsbeispiele mit Chinolin-, Pyridopyrazin- bzw. Indazolylresten werden jedoch nicht angeführt.

In der Patentschrift WO 00/67802 werden Indol-3-glyoxylamide, die mit höherkettigen Fettsäuren substituiert sind, als potentielle Antitumormittel beschrieben. Konkrete Ausführungsbeispiele mit Chinolin-, Pyridopyrazin- bzw. Indazolylresten werden jedoch nicht angeführt. Auch werden zu solchen Ausführungsbeispielen keine biologischen Daten angegeben.

In der Publikation von W.-T. Li et al. (J. Med. Chem. 2003, 46, 1706 ff.) werden N-Heterocyclische Indolylglyoxylamide als oral aktive Verbindungen mit antitumoraler Aktivität beschrieben. Angaben zum Wirkmechanismus werden jedoch nicht gemacht.

In der Patentanmeldung WO03/022280 A2 werden 3-Glyoxylamidindole und deren Verwendung als Arzneimittel für die Anti-Tumorbehandlung beschrieben. Unter deren allgemeine Formel fallen unter anderem 6-Chinolin-Derivate. Darüber hinaus werden zwei Beispiele mit 6-Chinolinrest als Ausführungsbeispiele benannt und mit biologischen Ergebnissen belegt. Konkrete Ausführungsbeispiele mit Pyridopyrazin- bzw. Indazolylresten werden jedoch nicht angeführt.

In der US-Anmeldung US03/0181482A1 werden neue Indolylgloxylamide beschrieben. Die erfindungsgemäßen Verbindungen werden hierbei als Antitumormittel mit cytotoxischer Aktivität und als Angiogenese-Inhibitoren beschrieben. Darüber hinaus wird ein 6-Chinolinderivat als Ausführungsbeispiel (Verbindung 3; S.10) gezeigt und mit antiproliferativen Daten (s. S.19; Table 1 a, 1 b) und antiangiogenetischen Eigenschaften (s. S. 20) belegt. Konkrete Ausführungsbeispiele mit Pyridopyrazin- bzw. Indazolylresten werden jedoch nicht angeführt.

Die WO 02/10152 A2 der Anmelderin beschreibt bereits eine andere Klasse von Indol-Derivaten für die Behandlung von Tumoren. Unter anderem wurde hier der Wirkstoff N-(2-Methyl-6-chinolyl)-[1-(4-chlorbenzyl)-indol-3-yl]-glyoxylsäureamid auf seine anti-proliferative Wirkung an verschiedenen Tumor-Zelllinien getestet.

Klinisch erprobte Verbindungen, die entweder an die Mikrotubuli binden (Paclitaxel, Vincristine) oder die Topoisomerase II inhibieren (Doxorubicin, Etoposide, Mitoxantron), werden gegenwärtig in der Krebstherapie u.a. gegen Brust-, Eierstock-, Magen-, Lungenkrebs, beim Kaposi-Sarkom als auch bei Leukämien erfolgreich eingesetzt. Ihr Einsatz wird jedoch durch das Auftreten von Arzneimittel-Resistenzen als auch durch schwerwiegende neurologische, gastrointestinale, cardiovaskuläre und hepatische Nebenwirkungen limitiert.

Eine der Erfindung zugrundeliegende Aufgabe ist es nun, zytotoxische Substanzen mit kombinierten Wirkmechanismen zur Verfügung zu stellen, die sich zur Behandlung einer Vielzahl von Tumoren, insbesondere bei Wirkstoffresistenzen gegen andere Arzneimittel und bei metastasierenden Karcinomen eignen.

Diese Aufgabe wird gelöst durch Indol-Derivate der allgemeinen Formel I 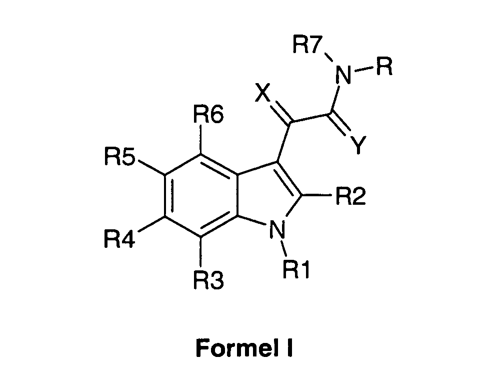 worin
- R:: für einen oder mehrere Heteroatome ausgewählt aus der Gruppe N, O und S enthaltenden gesättigten, ungesättigten oder aromatischen direkt mit dem Amidstickstoff verbundenen substituierten oder unsubstituierten (C₂-C₁₄)-Heterocyclus steht, wobei der Heterocyclus vorzugsweise
(i) unsubstituiertes oder substituiertes 5-,6-,7-Chinolyl,
(ii) unsubstituiertes oder substituiertes 2-,3-,6-, 7- und 8-Pyridopyrazinyl,
(iii) unsubstituiertes oder substituiertes 3-, 4-, 5-, 6- und 7-Indazolyl,
(iv) unsubstituiertes oder substituiertes 2-,3-,4-,5- und 6-Pyridyl,
(v) unsubstituiertes oder substituiertes 3-, 4- und 5-Isoxazolyl bedeutet,
(vi) unsubstituiertes oder substituiertes 3-, 4- und 5-Isothiazolyl bedeutet,
- R1:: unsubstituiertes oder substituiertes Alkyl-Aryl,
- R2:: (i) Wasserstoff,
(ii) unsubstituiertes oder substituiertes (C₁-C₆)-Alkyl,
- R3-R6:: (i) Wasserstoff
(ii) unsubstituiertes oder substituiertes (C₁-C₆)-Alkyl,
(iii) unsubstituiertes oder substituiertes (C₃-C₇)-Cycloalkyl,
(iv) Amino, Mono-(C₁-C₄)-Alkylamino, Di-(C₁-C₄)-Alkylamino,
(v) Halogen,
(vi) mit ein oder mehreren Fluoratomen substituiertes (C₁-C₄)-Alkyl, vorzugsweise Trifluormethylgruppe,
(vii) Cyano, geradkettiges oder verzweigtes Cyano-(C₁-C₆)-Alkyl,
(viii) (C₁-C₆)-Alkylcarbonyl,
(ix) Carboxyl, (C₁-C₄)-Alkoxycarbonyl, Carboxy-(C₁-C₆)-Alkyl oder (C₁-C₆)-Alkoxycarbonyl-(C₁-C₆)-Alkyl,
(x) Hydroxy,
(xi) -(C₁-C₆)-Alkoxy,
(xii) Aryl-(C₁-C₄)-Alkoxy, vorzugsweise Benzyloxy,
(xiii) (C₁-C₆)-Alkoxycarbonylamino, (C₁-C₆)-Alkoxycarbonylamino-(C₁-C₆)-Alkyl,
- R7:: (C₁-C₆)-Alkylcarbonyl, vorzugsweise Acetyl, Propionyl, (C₁-C₆)-Alkoxycarbonyl, vorzugsweise Methoxycarbonyl, Ethoxycarbonyl, Propoxycarbonyl,
und
- X,Y:: Sauerstoff oder Schwefel bedeuten,
deren Tautomere, Stereoisomere, einschließlich der Diastereomere und Enantiomere, sowie die physiologisch verträglichen Salze davon.

Für den Fall, dass R für eine unsubstituierte oder substituierte 2-,3-,4-,5- oder 6-Pyridyl-Gruppe steht und R1-R6 o.g. Bedeutung hat, darf R7 in diesem Falle nicht für einen Acetyl-Rest oder eine *tert*-Butyloxycarbonylgruppe stehen.

Ein weiterer Gegenstand der Erfindung sind Indol-Derivate der Formel I, worin 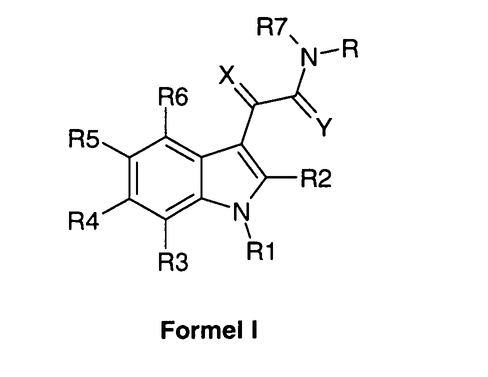
- R:: direkt mit dem Amidstickstoff verbundenes
(i) substituiertes 6-Chinolyl, unsubstituiertes oder substituiertes 7-Chinolyl, wobei 2-Methyl-6-Chinolyl ausgeschlossen ist, wobei wenn X ein Schwefelatom ist, R auch nicht substituiertes 6-Chinolyl sein kann.
(ii) unsubstituiertes oder substituiertes 2-,3-,6-, 7- und 8-Pyridopyrazinyl,
(iii) unsubstituiertes oder substituiertes 3-, 4-, 5-, 6- und 7-Indazolyl,
- R1:: unsubstituiertes oder substituiertes Alkyl-Aryl,
- R2:: Wasserstoff
- R3-R6:: (xiv) Wasserstoff
(xv) unsubstituiertes oder substituiertes (C₁-C₆)-Alkyl,
(xvi) unsubstituiertes oder substituiertes (C₃-C₇)-Cycloalkyl,
(xvii) Amino, Mono-(C₁-C₄)-Alkylamino, Di-(C₁-C₄)-Alkylamino,
(xviii) Halogen,
(xix) mit ein oder mehreren Fluoratomen substituiertes (C₁-C₄)-Alkyl, vorzugsweise Trifluormethylgruppe,
(xx) Cyano, geradkettiges oder verzweigtes Cyano-(C₁-C₆)-Alkyl,
(xxi) (C₁-C₆)-Alkylcarbonyl,
(xxii) Carboxyl, (C₁-C₄)-Alkoxycarbonyl, Carboxy-(C₁-C₆)-Alkyl oder (C₁-C₆)-Alkoxycarbonyl-(C₁-C₆)-Alkyl,
(xxiii) -(C₁-C₆)-Alkoxy,
(xxiv) Aryl-(C₁-C₄)-Alkoxy, vorzugsweise Benzyloxy,
(xxv) (C₁-C₆)-Alkoxycarbonylamino, (C₁-C₆)-Alkoxycarbonylamino-(C₁-C₆)-Alkyl
und
- R7:: Wasserstoff
- X, Y:: Sauerstoff oder Schwefel bedeuten,
deren Tautomere, Stereoisomere, einschließlich der Diastereomere und Enantiomere, sowie die physiologisch verträglichen Salze davon.

Die vorliegende Erfindung ist eine Weiterentwicklung der Erfindung, die in der WO 02/10152 beschrieben ist. Es wurde festgestellt, dass die Indol-Derivate bei Austausch der 2-Methyl-6-chinolyl-Gruppe gegen unsubstituiertes oder substituiertes 2-,3-,6-,7- und 8-Pyridopyrazinyl, unsubstituiertes oder substituiertes 3-, 4-, 5-, 6- und 7-Indazolyl, eine verbesserte antiproliferative Wirkung an verschiedenen Tumor-Zelllinien zeigen.

Es wurde weiterhin festgestellt, dass die erfindungsgemäßen Verbindungen eine starke zytotoxische Wirkung ausüben, die auf den unterschiedlichsten Mechanismen beruhen kann. Ein in der Erfindung nachgewiesener Mechanismus der erfindungsgemäßen Verbindungen beruht auf der Inhibierung der Tubulinpolymerisation und auf der Inhibierung der Topoisomerase II. Dies führt zu einer Zell-Arretierung von tumorigenen Zellen in der G2M-Phase. Darüber hinaus induzieren die erfindungsgemäßen Verbindungen Apoptose.

Es wurde weiterhin festgestellt, dass die erfindungsgemäßen Verbindungen eine verbesserte Wasserlöslichkeit und somit auch eine verbesserte orale Bioverfügbarkeit besitzen.

Ferner konnte für die erfindungsgemäßen Verbindungen eine verbesserte in vivo Aktivität bei zugleich besserer Verträglichkeit durch Einführung eines Acetyl-Restes als R7-Rest gezeigt werden.

Mit der in der Erfindung beschriebenen Stoffklasse soll die Möglichkeit einer niedrigeren, länger anhaltenden und besser verträglichen Medikation mit Antitumorwirkung eröffnet werden als mit den klassischen Cytostatika. Insbesondere soll die nachteilige Resistenzbildung, wie sie von vielen Antitumormitteln bekannt ist, umgangen werden. Die mit den erfindungsgemäßen Indolderivaten erzielte Wirkungsverstärkung soll den Arzneimittelverbrauch effektiver gestalten. Darüber hinaus sollte es möglich sein, die Behandlung auf therapieresistente Fälle auszudehnen.

In einer bevorzugten Ausführungsform bedeutet bei dem Indol-Derivat der Formel I R1 4-Chlorbenzyl, R2-R6 Wasserstoff, R Heterocyclus und R7 Alkylcarbonyl oder Alkoxycarbonyl.

In einer weiteren bevorzugten Ausführungsform bedeutet bei dem Indol-Derivat der Formel I R nicht substituiertes 5-Chinolyl, nicht substituiertes 6-Chinolyl oder nicht substituiertes 7-Chinolyl und R7 Acetyl oder Propionyl.

In einer weiteren bevorzugten Ausführungsform bedeutet bei dem Indol-Derivat der Formel I R nicht substituiertes 5-Chinolyl, nicht substituiertes 6-Chinolyl oder nicht substituiertes 7-Chinolyl und R7 Methoxycarbonyl, Ethoxycarbonyl oder Propionoxycarbonyl.

Nachfolgend werden einige Begriffe definiert, die in der Beschreibung und den Patentansprüchen verwendet werden.

Im Zusammenhang mit "Heterocyclus" versteht man unter dem Begriff, insofern oben nicht explizit genannt Pyrrol, Furan, Thiophen, Pyrazol, Thiazol, Indol, Oxazol, Imidazol, Isothiazol, Isoxazol, 1,2,3-Triazol, 1,2,4-Triazol, 1,2,4,Oxadiazol, 1,3,4-Oxadiazol, 1,2,5-Thiadiazol, 1,3,4-Thiadiazol, Tetrazol, Pyridin, Pyrimidin, Pyridazin, Pyrazin, Benzofuran, Indazol, Carbazol, Benzoxazol, Benzimidazol, Benzothiazol, Benzotriazol, Chinolin, Cinnolin, Chinoxalin, Chinazolin, Phthalazin, Pyridopyrazin, 1,2,3-Triazin, 1,2,4-Triazin, 1,3,5-Triazin, Purin, Pteridin, Acridin und Phenanthridin.

Der Ausdruck "Alkyl" umfasst im Sinne dieser Erfindung acyclische gesättigte oder ungesättigte Kohlenwasserstoffe, die geradkettig oder verzweigt sein können.
Im Zusammenhang mit "Alkyl" versteht man unter dem Begriff "substituiert" im Sinne dieser Erfindung, insofern nicht oben explizit definiert, die Substitution eines Wasserstoffrestes durch F, Cl, Br, I, CN, NH₂, NH-Alkyl, NH-Cycloalkyl, OH, O-Alkyl, wobei unter mehrfach substituierten Resten solche zu verstehen sind, die entweder an verschiedenen oder an gleichen Atomen mehrfach, z.B. zwei- oder dreifach substituiert sind, beispielsweise dreifach am gleichen C-Atom wie im Falle von -CF₃, -CH₂CF₃ oder an verschiedenen Stellen wie im Falle von -CH(OH)-CH₂-CH₂-CHCl₂. Die Mehrfachsubstitution kann mit dem gleichen oder verschiedenen Substituenten erfolgen.

Der Ausdruck "Alkyl-Aryl" bedeutet (C₁-C₆)-Alkyl-(C₆-C₁₄)-Aryl und vorzugsweise (C₁-C₆)-Alkyl-C₆-Aryl.
In Bezug auf "Alkyl-Aryl" sowie auf "Cycloalkyl" versteht man im Sinne dieser Erfindung- insofern oben nicht explizit erwähnt unter "ein- oder mehrfach substituiert" die ein- oder mehrfache z.B. zwei-, drei- oder vierfache Substitution eines oder mehrerer Wasserstoffatome des Ringsystemes durch F, Cl, Br, I, CN, NH₂, NH-Alkyl, OH, O-Alkyl, CF₃, Alkyl, (C₆-C₁₀)-Aryl, (C₆-C₁₀)-Aryl-(C₁-C₆)-alkyl und/oder Heterocyclyl, an einem oder ggf. verschiedenen Atomen (wobei ein Substituent ggf. seinerseits substituiert sein kann). Die Mehrfach-Substitution erfolgt dabei mit dem gleichen oder mit unterschiedlichen Substituenten.

In Bezug auf "Heterocyclus" versteht man im Sinne dieser Erfindunginsofern oben nicht explizit erwähnt unter "ein- oder mehrfach substituiert" die ein- oder mehrfache z.B. zwei-, drei- oder vierfache Substitution eines oder mehrerer Wasserstoffatome des Ringsystemes durch F, Cl, Br, I, Nitro, Amino, C₁-C₆-Alkyl, vorzugsweise Methyl, Mono-(C₁-C₆)-alkylamino, Di-(C₁-C₆)-alkylamino, Hydroxy, C₁-C₆-Alkoxy, Benzyloxy, Carboxy, (C₁-C₆)-Alkoxycarbonyl, (C₁-C₆)-Alkoxycarbonylamino oder ein- oder mehrfach mit Fluor substituiertem (C₁-C₆)-Alkyl, vorzugsweise Trifluormethyl, (C₆-C₁₀)-Aryl und/oder (C₆-C₁₀)-Aryl-(C₁-C₆)-alkyl an einem oder ggf. verschiedenen Atomen (wobei ein Substituent ggf. seinerseits substituiert sein kann). Die Mehrfach-substitution erfolgt dabei mit dem gleichen oder mit unterschiedlichen Substituenten.

Sofern die erfindungsgemäßen Verbindungen der allgemeinen Formel I mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomere vorliegen. Sofern möglich, können die erfindungsgemäßen Verbindungen in Form der Tautomeren vorliegen.

So lassen sich beispielsweise die erfindungsgemäßen Verbindungen gemäß der allgemeinen Formel I, welche ein oder mehrere Chiralitätszentren aufweisen und die als Racemate auftreten, nach an sich bekannten Methoden in ihre optischen Isomere, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Die erfindungsgemäßen Verbindungen der allgemeinen Formel **I** können, falls sie eine ausreichend saure Gruppe, wie zum Beispiel die Carboxygruppe enthalten, mit anorganischen und/oder organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen der allgemeinen Formel **I** können, falls sie eine ausreichend basische Gruppe, wie zum Beispiel ein sekundäres oder tertiäres Amin besitzen, mit anorganischen und organischen Säuren in Salze überführt werden. Vorzugsweise werden die pharmazeutisch annehmbaren Salze der erfindungsgemäßen Verbindungen gemäß der allgemeinen Struktur I mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Phosphate, Methansulfonate, Sulfoessigsäure, Tosylate, Carbonate, Hydrogencarbonate, Formiate, Acetate, Triflate, Oxalate, Malonate, Maleate, Succinate, Tartrate, Malate, Embonate, Mandelate, Fumarate, Lactate, Citrate und Glutaminate. Die Stöchiometrie der gebildeten Salze der erfindungsgemäßen Verbindungen kann dabei ganzzahlige oder nicht ganzzahlige Vielfache von eins betragen.

Ebenfalls bevorzugt sind Solvate und insbesondere Hydrate der erfindungsgemäßen Verbindungen **I**, die z. B. durch Kristallisation aus einem Lösungsmittel oder aus wässriger Lösung erhalten werden können. Es können sich dabei ein, zwei, drei oder beliebig viele Solvat- oder Wasser-Moleküle mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen der allgemeinen Formel **I** können in verschiedenen polymorphen Formen vorliegen, dabei können bestimmte Modifikationen metastabil sein.

Sowohl die Verbindungen der Formel **I** als auch deren Salze sind biologisch aktiv. Die Verbindungen der Formel **I** können in freier Form oder als Salze mit physiologisch verträglichen Säuren oder Basen verabreicht werden.

Die Verabreichung der Verbindungen der allgemeinen Formel kann oral, rectal, buccal (z.B. sublingual), parenteral (z.B. subkutan, intramuskulär, intradermal oder intravenös), topisch oder transdermal erfolgen.

Weiterhin betrifft die Erfindung Arzneimittel mit einem Gehalt mindestens eine der Verbindungen der Formel I oder deren Salze mit physiologisch verträglichen anorganischen oder organischen Säuren und gegebenenfalls pharmazeutisch verwendbaren Träger- und/oder Verdünnungs- bzw. Hilfsstoffen.

Diese Arzneimittel werden zur Behandlung von Tumorerkrankungen, insbesondere zur Behandlung bei Tumorerkrankungen mit Arzneimittelresistenz gegen andere Wirkstoffe und/oder bei Tumorerkrankungen mit metastasierendem Karcinom eingesetzt.

Als Applikationsformen eignen sich beispielsweise Tabletten, Dragees, Kapseln, Lösungen zur Infusion oder Ampullen, Suppositorien, Pflaster, inhalativ einsetzbare Pulverzubereitungen, Suspensionen, Cremes und Salben.

Die erfindungsgemäßen Verbindungen können auch in einer mikropartikulären z. B. nanopartikulären Zusammensetzung dispergiert sein.

Die gefundenen, therapeutisch wertvollen Eigenschaften beziehen sich im einzelnen auf die nachfolgenden Vorteile:
- die erfindungsgemäßen Verbindungen zeichnen sich durch starke antiproliferative Eigenschaften aus;
- die erfindungsgemäßen Verbindungen inhibieren die Tubulin-Polymerisation;
- die erfindungsgemäßen Verbindungen inhibieren die Topoisomerase II;
- die erfindungsgemäßen Verbindungen arretieren sich teilende Zellen in der G2/M-Phase;
- die erfindungsgemäßen Verbindungen induzieren Apoptose;
- die erfindungsgemäßen Verbindungen zeichnen sich durch starke antitumorale in vivo-Aktivitäten bei zudem verbesserten Verträglichkeiten aus;
- die erfindungsgemäßen Verbindungen der Formel I sind in vitro an mdrresistenten Zell-Linien im Gegensatz zu Paclitaxel, Vincristin, Doxorubicin oder Etoposide aktiv;

Am meisten bevorzugt sind Verbindungen gemäß der allgemeinen Formel I, die in der folgenden Auswahl getroffen sind:
2-[1-(4-Chlor-benzyl)-1H-indol-3-yl]-2-oxo-N-pyrido[2,3-b]pyrazin-7-yl-acetamid **(1)**
2-[1-(4-Chlor-benzyl)-1H-indol-3-yl]-N-(1H-indazol-5-yl)-2-oxo-acetamid (4)
N-{2-[1-(4-Chlor-benzyl)-1H-indol-3-yl]-2-oxo-acetyl}-N-chinolin-6-yl-acetamid **(2)**
{2-[1-(4-Chlor-benzyl)-1*H*-indol-3-yl]-2-oxo-acetyl}-chinolin-6-yl-carbaminsäure-methylester **(3)**
{2-[1-(4-Chlor-benzyl)-1*H*-indol-3-yl]-2-oxo-acetyll-chinolin-6-yl-carbaminsäure-ethylester **(5)**
{2-[1-(4-Chlor-benzyl)-1*H*-indol-3-yl]-2-oxo-acetyl}-chinolin-6-yl-carbaminsäure- propylester **(6)**
*N*-{2-[1-(4-Chlor-benzyl)-1*H*-indol-3-yl]-2-oxo-acetyl}-*N*-chinolin-6-yl-propionamid **(7)**
{2-[1-(4-Chlor-benzyl)-1*H*-indol-3-yl]-2-oxo-acetyl}-pyridin-4-yl-carbaminsäure-ethylester **(8)**
2-[1-(4-Chlor-benzyl)-1H-indol-3-yl]-N-chinolin-6-yl-2-thioxo-acetamid **(11)**

Die Verbindungen (1), (4) und (11) sind Verbindungen, wobei der Rest R7 Wasserstoff bedeutet. Die Verbindungen (2), (3), (5) und (6) bis (8) enthalten als Gruppe R7 eine Alkylcarbonyl- oder Alkoxycarbonylgruppe.

Die nachfolgenden Verbindungen (9), (10), (12), (13), (14) und (15) sind Verbindungen, die zu Vergleichszwecken mit untersucht wurden. Die Verbindungen (9), (10), (14) und (15) sind aus dem Stand der Technik bekannt. Verbindung (9) ist in WO02/10152 der Anmelderin beschrieben, Verbindung (10) in der WO03/022280, Verbindung (13) fällt unter die Ansprüche der WO02/08225 A1, die Verbindungen (12), (14) und (15) fallen unter die Ansprüche von WO99/51224A1 und WO01/22954.
2-[1-(4-Chlor-benzyl)-1H-indol-3-yl]-N-(2-methyl-chinolin-6-yl)-2-oxo-acetamid **(9)**
2-[1-(4-Chlor-benzyl)-1H-indol-3-yl]-2-oxo-N-chinolin-6-yl-acetamid **(10)**
2-[1-(4-Chlor-benzyl)-1H-indol-3-yl]-2-oxo-N-chinolin-8-yl-acetamid **(12)**
2-[1-(4-Chlor-benzyl)-1H-indol-3-yl]-N-isochinolin-5-yl-2-oxo-acetamid **(13)**
2-[1-(4-Chlor-benzyl)-1H-indol-3-yl]-2-oxo-N-pyridin-4-yl-acetamid **(14)**
2-[1-(4-Fluor-benzyl)-1H-indol-3-yl]-N-(2-methyl-chinolin-8-yl)-2-oxo-acetamid **(15)**

Die Verbindungen der allgemeinen Formel **Ia** und **Ib** des Schemas sind gemäß des folgenden Schemas 1 erhältlich:

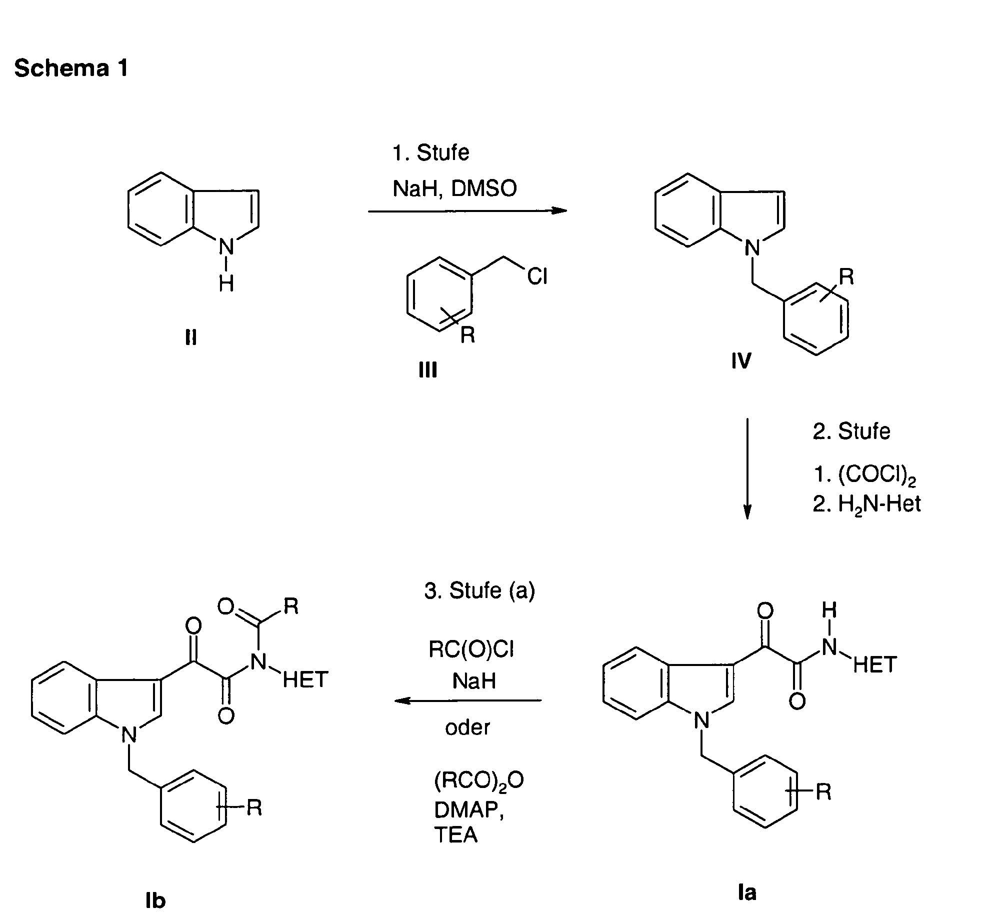

Die Verbindungen der allgemeinen Formel Ic mit X=S können nach Schema 2 hergestellt werden:

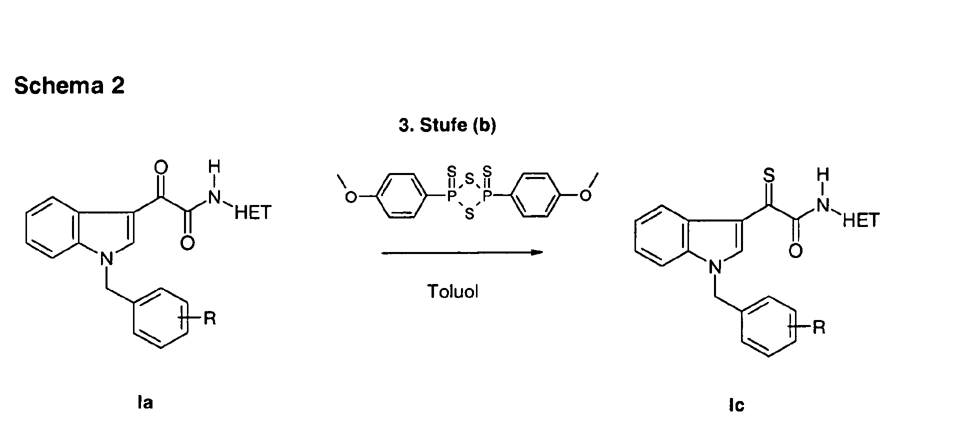

Verbindungen der allg. Formel **Ic** mit Y = S können nach Literatur bekannten Methoden erhalten werden (W.-D. Malmberg et al. Liebigs Ann. Chem. 10, 1983; 1649-1711).

Die Ausgangsverbindungen **II**, **III** und **IV** sind entweder im Handel erhältlich oder können nach an sich bekannten Verfahrensweisen hergestellt werden. Die Edukte **II**, **III** und **IV** stellen wertvolle Zwischenverbindungen für die Herstellung der erfindungsgemäßen Indolderivate der Formel **I** dar.

Für die Herstellung der Ausgangs- und Zielverbindungen sei beispielsweise auf folgende Standardwerke der organischen Synthese verwiesen, dessen Inhalt hiermit Bestandteil der Offenbarung der vorliegenden Anmeldung werden soll:
- Houben-Weyl, Band E 7a (Teil1) S. 290-492, S.571-740
- Houben-Weyl, Band E 7a (Teil 2) S. 119-156, S.205-686, S. 157-204
- Monographie "Heterocyclic Compounds" (Elderfield),
   Volume 1, S.119- 207, S. 397-616
   Volume 3, S. 1-274
   Volume 6, S. 101-135, S. 234-323
- Monographie "Comprehensive Organic Chemistry" (S.D.Barton, W.D. Ollis)
   Volume 4, S.155-204, S.205-232, S.493-564

Die gegebenenfalls zu verwendenden Lösungs- und Hilfsmittel und anzuwendenden Reaktionsparameter wie Reaktionstemperatur und -dauer sind dem Fachmann aufgrund seines Fachwissens bekannt.

Gemäß dieser allgemeinen Vorschrift für die Stufen 1, 2 und 3, denen das Syntheseschemata 1 und 2 zugrunde liegt, wurden folgende Verbindungen synthetisiert, die unter der Angabe der jeweiligen chemischen Bezeichnung aus der nachfolgenden Übersicht hervorgehen. Die analytische Charakterisierung der erfindungsgemäßen Verbindungen erfolgte durch ihre Schmelzpunkte bzw. 1 H-NMR-spektroskopisch und/oder massenspektroskopisch.

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.

Die Erfindung soll anhand der nachfolgenden Beispiele näher erläutert werden, ohne darauf beschränkt zu sein.

### Beispiele

### Beispiel 1 (Umsetzung gemäß Schema 1, 1. Stufe):

### Herstellung von 1-(4-Chlorbenzyl)-indol

In eine Mischung von 1,32 g Natriumhydrid (0,055 Mol, Mineralölsuspension) in 50 ml Dimethylsulfoxid wird eine Lösung von 5,86 g (0,05 Mol) Indol in 25 ml DMSO gegeben. Man erhitzt 1,3 Stunden auf 60°C, läßt danach abkühlen und tropft 17,7 g (0,11 Mol) 4-Chlor-benzylchlorid zu. Man erwärmt die Lösung auf 60°C, läßt über Nacht stehen und gießt sodann unter Rühren in 200 ml Wasser. Man extrahiert mehrmals mit insgesamt 75 ml CH₂Cl₂, trocknet die organische Phase mit wasserfreiem Natriumsulfat, filtriert und engt das Filtrat im Vakuum ein. Ausbeute: 11,5g (95% d. Th.)

### Beispiel 2 (Umsetzung gemäß 2. Stufe von Schema 1):

### 2-[1-(4-Chlor-benzyl)-1H-indol-3-yl]-2-oxo-N-pyrido[2,3-b]pyrazin-7-yl-acetamid (1)

Zu einer Lösung von 1,12 ml Oxalylchlorid in 50 ml Ether wird bei 0°C und unter Stickstoff tropfenweise eine Lösung von 10,2 g (10,7 mmol) 1-(4-Chlorbenzyl)-indol in 200 ml Ether gegeben. Man erhitzt 2 Std. zum Rückfluss und dampft anschließend das Lösungsmittel ab. Sodann wurden zum Rückstand 30 ml DMF zugefügt, 1,93 g (13,9 mMol) Kaliumcarbonat hinzugegeben, die Suspension auf 0°C gekühlt und tropfenweise mit einer Lösung von 1,57 g (10,7 mmol) Aminkomponente in 10 ml DMF versetzt. Man lässt über Nacht bei Raumtemperatur rühren. Das Reaktionsgemisch wird schliesslich in Eiswasser eingerührt und der entstehende Niederschlag abgesaugt. Das erhaltene Rohprodukt wird an 100 g Kieselgel mit n-Heptan/ Essigester = 4:1 chromatographiert.
Ausbeute: 3,23 g (68,0%)
Fp.: 250°C
1 H-NMR (DMSO-D6) δ= 11,56 (s, 1 H), 9,53 (d, 1 H), 9,12 (s, 1 H), 9,09 (d, 1 H), 9,04 (s, 1 H), 8,32 (d, 1 H), 7,6 (d, 1 H), 7.40 (d, 2H), 7,35 (m, 3H), 7.32 (m, 2H) 5,64 (s, 2H) ppm

### Beispiel 3 (Umsetzung gemäß 3. Stufe (a) von Schema 1):

### N-{2-[1-(4-Chlor-benzyl)-1H-indol-3-yl]-2-oxo-acetyl}-N-chinolin-6-yl-acetamid (2)

Zu einer gerührten Lösung von 6,0 g (13.6 mmol) 2-[1-(4-Chlorbenzyl)-1 H-indol-3-yl]-2-oxo-N-chinolin-6-yl-acetamid in 60 ml DMF werden unter Stickstoff 0,833 g (6,82 mmol) DMAP, 1,38 g (13,6 mmol) Triethylamin und 13.9 g (136 mmol) Essigsäureanhydrid gegeben. Die Reaktionsmischung wird bei Raumtemperatur 10 Minuten gerührt und danach in 200 ml Ethylacetat gegossen. Nach Zugabe von 300 ml Wasser wird die Mischung in einem Scheidetrichter geschüttelt, wonach sich beide Phasen trennen. Die Ausfällung beginnt nach 20 Minuten. Die hellgelben Kristalle werden abfiltriert und im Vakuum bei 60°C getrocknet.
Ausbeute: 4,04 g (61,5%)
Fp: 122,9 °C
¹H NMR (600 MHz, DMSO-d6) δ= 9.02 (d, 1 H), 8.54 (s, 1 H), 8.44 (d, 1 H), 8.21 (d, 1 H), 8.17 (d, 1 H), 8.10 (m, 1 H), 7.88 (m, 1 H), 7.65 (m, 1 H), 7.58 (m, 1 H), 7.44 (d, 2 H), 7.33 (d, 2 H), 7.28 (m, 2 H), 5.60 (s, 2 H), 2.15 (s, 3 H).
MS (ESI) m/z 482.1 (MH⁺), (theor. 481.94)

### Beispiel 4 (Umsetzung gemäß 3. Stufe (a) von Schema 1):

### {2-[1-(4-Chlor-benzyl)-1H-indol-3-yl)-2-oxo-acetyl}-chinolin-6-yl-carbaminsäure-methylester (3)

Zu einer gekühlten, gerührten Lösung von 10,0 g (22,7 mmol) 2-[1-(4-Chlorbenzyl)-1H-indol-3-yl]-2-oxo-N-chinolin-6-yl-acetamid in 500 ml trockenem THF werden unter Stickstoff 930,2 mg (27,3 mmol) NaH (als 60%ige Dispersion in Mineralöl) gegeben. Die Lösung wird bei 0°C gerührt, bis ein gelber Niederschlag ausfällt und danach weitere 15 Minuten gerührt. Danach werden 2,58 g (27,3 mmol) Methylchlorformiat tropfenweise bei einer Temperatur unter +5°C zugefügt. Die Reaktion wird dünnschichtchromatographisch verfolgt (Eluent: n-Heptan/Ethylacetat 1/1 RF=0.11). Die Reaktionsmischung wird in Wasser gegossen, mit Ethylacetat extrahiert, die organische Phase mit gesättigter Natriumchloridlösung gewaschen und über wasserfreiem MgSO4 getrocknet. Die Verdampfung des Lösungsmittels ergibt ein Rohprodukt, das durch Säulenchromatographie (n-Heptan/Aceton 2/1) zu **3** gereinigt wird. **3** zeigt noch geringe Verunreinigungen in der Dünnschichtchromatographie, die durch Ausrühren des rohen **3** über 1 h mit Aceton entfernt werden können. Die Filtration ergibt 3 als hellgelbe Kristalle.
Ausbeute: 3,0 g (26,5%)
Fp: 178,5 °C
¹H NMR (600 MHz, DMSO-d6) δ= 9.02 (d, 1 H), 8.58 (s, 1 H), 8.47 (d, 1 H), 8.17 (m, 3 H), 7.84 (m, 1 H), 7.63 (m, 2 H), 7.44 (d, 2 H), 7.34 (m, 4 H), 5.60 (s, 2 H), 3.65 (s, 3 H).
MS (ESI) m/z 498.2 (MH⁺), (theor. 497.94)

### Beispiel 5 (Umsetzung gemäß 3. Stufe (b) von Schema 2):

### Herstellung von 2-[1-(4-Chlorbenzyl)-1 H-indol-3-yl]-N-chinolin-6-yl-2-thioxo-acetamid (11)

Eine Suspension von 4,00 g (9,1 mmol) 2-[1-(4-Chlorbenzyl)-1 H-indol-3-yl]-2-oxo-N-chinolin-6-yl-acetamid in 200 ml Toluol wird unter Stickstoff mit 3,68 g (9,1 mmol) 2,4-Bis-(4-methoxyphenyl)-1,3-dithia-2,4-diphosphetan-2,4-disulfid versetzt und anschließend für 3 h auf 75 °C erwärmt. Die Reaktionslösung wird heiß vom entstandenen Rückstand abfiltriert und dieser mit 100 ml Methylenchlorid nachgewaschen. Das Filtrat wird i. Vak. eingeengt und der Rückstand an Flashkieselgel chromatographiert (Eluent: Methylenchlorid/Methanol 99 : 1). Die Produktfraktionen werden nach erneutem Entfernen des Lösungsmittels i. Vak. an Flashkieselgel filtriert (Eluent: n-Heptan/Ethylacetat **1** : 1).
Ausbeute: 0,46 g (11 % d. Th.)
ESI-MS: m/e = 456.1 (MH+), (theor. 455.97)
1 H-NMR (DMSO-D6) δ= 10,89 (s, 1 H), 8,8 (s, 1 H), 8,75 (s, 1 H), 8,55 (s, 1 H), 8,12 (d, 1 H), 8,35 (d, 1 H), 8,0 (d, 1 H), 7.93 (d, 1 H), 7.63 (d, 1 H), 7.50 (m, 1 H), 7,4 (m, 3H), 7,3 (m, 3H), 5,6 (s, 2H) ppm.

Folgende Verbindungen der Formel **I** wurden analog zum Syntheseweg in Schema 1 und gemäß den Beispielen 2 und 3 synthetisiert.

### Beispiel 6:

### 2-[1-(4-Chlor-benzyl)-1H-indol-3-yl]-N-(1H-indazol-5-yl)-2-oxo-acetamid (4)

Fp.: 203°C
¹H-NMR (DMSO-D₆) δ= 13,02 (s, 1 H), 10,7 (s, 1 H), 9,04 (s, 1 H), 8,48 (s, 1 H), 8,42 (d, 1 H), 8,06 (s, 1 H), 7,73 (d, 1 H), 7,6 (d, 1 H), 7.55 (d, 1 H), 7.40 (d, 2H), 7.28-7,35 (m, 4H), 5,63 (s, 2H) ppm

### Beispiel 7:

### {2-[1-(4-Chlor-benzyl)-1H-indol-3-yl]-2-oxo-acetyl}-chinolin-6-yl-carbaminsäure-ethylester (5)

Fp: 199 °C
¹H NMR (600 MHz, DMSO-d6) δ= 9.02 (m, 1 H), 8.60 (s, 1 H), 8.48 (d, 1 H), 8.15 (m, 3 H), 7.83 (m, 1 H), 7.63 (m, s H), 7.43 (d, 2 H), 7.32 (m, 4 H), 5.60 (s, 2 H), 4.15 (q, 2 H), 0.95 (t, 3 H).
MS (ESI) m/z 514.2, 512.1 (MH⁺), (theor. 511.97)

### Beispiel 8:

### {2-[1-(4-Chlor-benzyl)-1H-indol-3-yl]-2-oxo-acetyl}-chinolin-6-yl-carbaminsäure-propylester (6)

Fp: 164°C
¹H NMR (600 MHz, DMSO-d6) δ= 9.02 (m, 1 H), 8.60 (s, 1 H), 8.48 (d, 1 H), 8.17 (m, 3 H), 7.84 (m, 1 H), 7.63 (m, 2 H), 7.43 (d, 2 H), 7.33 (m, 4 H), 5.61 (s, 2 H), 4.03 (t, 2 H), 1.32 (m, 2 H), 0.56 (t, 3 H).

### Beispiel 9:

### N-{2-[1-(4-Chlor-benzyl)-1 H-indol-3-yl]-2-oxo-acetyl}-N-chinolin-6-yl-propionamid (7)

¹H NMR (600 MHz, DMSO-d6) δ= 9.03 (m, 1 H), 8.52 (s, 1 H), 8.45 (d, 1 H), 8.23 (d, 2 H), 8.18 (d, 1 H), 8.13 (m, 1 H), 7.88 (m, 1 H), 7.65 (m, 1 H), 7.58 (m, 1 H), 7.45, (d, 2 H), 7.30 (m, 4 H), 5.59 (s, 2 H), 2.61 (q, 3 H), 0.88 (t, 3 H).

### Beispiel 10:

### {2-[1-(4-Chloro-benzyl)-1H-indol-3-yl]-2-oxo-acetyl}-pyridin-4-yl-carbaminsäure ethyl ester (8)

Fp: 62 °C
¹H NMR (500 MHz, DMSO-d6) δ= 8.74 (m, 2 H), 8.52 (s, 1 H), 8.12 (m, 1 H), 7.60 (m, 1 H), 7.55 (m, 2 H), 7.40 (m, 2 H), 7.30 (m, 4 H), 5.57 (s, 2 H), 4.10 (q, 2 H), 0.95 (t, 3 H).

### Beispiel 11 (Vergleichssubstanz):

### Herstellung von 2-[1-(4-Chlor-benzyl)-1H-indol-3-yl]-N-(2-methyl-chinolin-6-yl)-2-oxo-acetamid (9)

Ausbeute: 14,8 g (77,3% d.Th.)
Fp: 182-185°C
¹H-NMR (CDCl₃) δ= 9,58 (s, 1 H), 9,12 (s, 1 H), 8,5 (s, 1 H), 8,41 (s, 1 H), 8,05 (t, 2H), 7.78 (d, 1 H), 7.4 (dd, 1 H), 7.32 (m, 4H), 7.26 (s, 1 H), 7.15 (d, 1 H), 5,38 (s, 2H), 2,73 (s, 3H) ppm

### Beispiel 12 (Vergleichssubstanz):

### 2-[1-(4-Chlor-benzyl)-1 H-indol-3-yl]-2-oxo-N-chinolin-6-yl-acetamid (10)

Fp.: 200°C
¹H-NMR (DMSO-D₆) δ= 11,5 (s, 1 H), 9,05 (s, 1 H), 8,85 (s, 1 H), 8,66 (s, 1 H), 8,32 (d, 2H), 8,12 (d, 1 H), 8,03 (d, 1 H), 7.63 (d, 1 H), 7.53 (dd, 1 H), 7.42 (d, 2H), 7.30-7,38 (m, 4H), 5,63 (s, 2H) ppm

### Beispiel 13 (Vergleichssubstanz):

### 2-[1-(4-Chlor-benzyl)-1H-indol-3-yl]-2-oxo-N-chinolin-8-yl-acetamid (12)

Fp.: 178°C

### Beispiel 14 (Vergleichssubstanz):

### 2-[1-(4-Chlor-benzyl)-1H-indol-3-yl]-N-isochinolin-5-yl-2-oxo-acetamid (13)

Fp.: 239-241 °C

### Beispiel 15 (Vergleichssubstanz):

### 2-[1-(4-Chlor-benzyl)-1H-indol-3-yl]-2-oxo-N-pyridin-4-yl-acetamid (14)

Fp.: 264°C

### Beispiel 16 (Vergleichssubstanz):

### 2 [1-(4-Fluor-benzyl)-1H-indol-3-yl]-N-(2-methyl-chinolin-8-yl)-2-oxo-acetamid (15)

Fp.: 200-202°C

### Biologische Wirkungen der erfindungsgemäßen Verbindungen

Die in-vitro und in-vivo Testung an ausgewählten Tumormodellen ergab die nachfolgenden pharmakologischen Aktivitäten.

### Beispiel 17: Antiproliferative Wirkung an verschiedenen Tumorzelllinien

Die Substanzen **1, 2, 4, 9**, **11**, **12** , **13** und **15** wurden in einem Proliferationstest an etablierten Tumorzelllinien auf ihre anti-proliferative Aktivität hin untersucht (D.A. Scuderio et al. Cancer Res. 1988, 48, 4827-4833). Der verwendete Test bestimmt die zelluläre Dehydrogenase-Aktivität und ermöglicht eine Bestimmung der Zellvitalität und indirekt der Zellzahl. Bei den verwendeten Zelllinien handelt es sich um die humane Cervixkarzinom Zelllinie KB/HeLa (ATCC CCL17), die ovariale Adenokarzinomzelllinie SKOV-3 (ATCC HTB77), die humane Glioblastom Zelllinie SF-268 (NCI 503138) und die Lungenkarzinom Zelllinie NCI-H460 (NCI 503473).

**Tabelle 1:**

| Proliferationshemmung der erfindungsgemäßen Substanzen im XTT Zytotoxizitätstest an humanen Tumorzelinien | | | | |
|---|---|---|---|---|
| | XTT Proliferationsassay, EC50 in µg/ml | | | |
| **Beispiel** | **KB/Hela** | **SKOV3** | **SF-268** | **NCl-H460** |
| **1** | 0.045 | 0.029 | 0.042 | 0.046 |
| **2** | 0.202 | 0.123 | 0.166 | 0.168 |
| **4** | 0.335 | 0.144 | >3.16 | 0.233 |
| **11** | 0.036 | 0.029 | 0.036 | 0.057 |
| **9 (V)** | 0.183 | 0.174 | 0.261 | 0.344 |
| **12 (V)** | >3.16 | >3.16 | >3.16 | >3.16 |
| **13 (V)** | >3.16 | >3.16 | >3.16 | >3.16 |
| **15 (V)** | >3.16 | n.b. | >3.16 | n.b. |
| V = Vergleichssubstanz; n.b.: nicht bestimmt | | | | |

Die Ergebnisse zeigen eine sehr potente Hemmung der Proliferation ausgewählter Tumorzelllinien durch die Ausführungsbeispiele **1, 2, 4** und **11**.

### Beispiel 18: Antiproliferative Wirkung an MDR Tumorzelllinien

Zur weiteren Charakterisierung wurden die Substanzen 1, 2, 4 und 11 gegenüber multi drug resistenten Zelllinien im Vergleich zu den nicht-resistenten Wildtypzelllinien untersucht.

Bei den untersuchten Zelllinien handelt es sich um die murine L1210, die akute myeloische Leukaemie Zelllinie LT12 und um die resistenten Linien L1210/mdr und LT12/mdr. Außerdem wurden die murine P388 Zelllinie (methyl-cholanthreneinduced lymphoid neoplasm) sowie die Doxorubicin-resistente P388 als Testsysteme herangezogen.

In der nachfolgenden Tabelle 2 sind die Ergebnisse zusammengefasst dargestellt:

**Tabelle 2:**

| Hemmwirkung der Substanzen im XTT Proliferationstest an humanen Tumorzellinien. | | | | | | |
|---|---|---|---|---|---|---|
| XTT Proliferationsassay, EC50 in *µ*g/ml | | | | | | |
| **Beispiel** | **LT12** | **LT12mdr** | **L1210** | **L1210VCR** | **P388** | **P388ADR** |
| **1** | 0.015 | 0.017 | 0.018 | 0.021 | 0.012 | 0.019 |
| **2** | 0.225 | 0.272 | 0.206 | 0.558 | 0.224 | 0.215 |
| **4** | 0.084 | 0.093 | 0.246 | 0.241 | 0.175 | 0.231 |
| **11** | 0.023 | 0.054 | 0.052 | 0.067 | 0.018 | 0.051 |
| **Paclitaxel** | 0.005 | 0.34 | 0.048 | >3.16 | 0.035 | >3.16 |
| **Vincristine** | 0.002 | 0.134 | 0.015 | >3.16 | 0.004 | 0.93 |
| **Doxorubicin** | 0.029 | >3.16 | 0.269 | >3.16 | 0.204 | >3.16 |
| **Mitoxantron** | 0.006 | 3.1 | 0.09 | 2.1 | 0.053 | 0.608 |
| **Etoposide** | 0.094 | >3.6 | 0.269 | >3.16 | 0.202 | >3.16 |
| V = Vergleichsbeispiel | | | | | | |

Die Substanzen 1, 2, 4 und 11 zeigen eine sehr potente Hemmwirkung an allen getesteten Zelllinien während bei den klassischen tubulin-inhibitorisch wirksamen Substanzen wie Paclitaxel oder Vincristin ferner bei den Topoisomerase II-Inhibitoren (Doxorubicin, Mitoxantron, Etoposide) eine zumindest stark verminderte Wirkung an den MDR1 resistenten Zelllinien zu erkennen ist.

### Beispiel 19: Inhibierung der Polymerisation von Tubulin

Die Substanzen **1, 4, 9, 11, 12, 13** und **15** wurden in einem in-vitro Test auf Hemmung der Polymerisation von Rindertubulin getestet (D.M. Bollag et al. Cancer Res. 1995, 55, 2325-2333). In diesem Test wird durch Zyklen von Polymerisation und Depolymerisation aufgereinigtes Tubulin eingesetzt, welches durch Zugabe von GTP und Erwärmung zur Polymerisation gebracht wird. In Tabelle 3 sind die EC₅₀ Werte der Polymerisationshemmung von Tubulin mit 30% assoziierten Proteinen (MAPs) angegeben.

**Tabelle 3:**

| Hemmung der Tubulin-Polymerisation. Durchschnittswert aus zwei unabhängigen Versuchen. | |
|---|---|
| **Beispiel** | **Hemmung der Tubulin-Polymerisation, EC50 in µg/ml** |
| **1** | 0.71 |
| **4** | 1.26 |
| **11** | 0.97 |
| **9 (V)** | 1.16 |
| **12 (V)** | >10µM |
| **13 (V)** | >10*µ*M |
| **15 (V)** | >10*µ*M |
| Vincristine | 0.35 |
| V = Vergleichsbeispiel | |

Die Ergebnisse (s. Tabelle 3) zeigen eine sehr potente Hemmwirkung der Substanzen **1**, **4**, **9** und **11** auf die Polymerisation von Tubulin während die Verbindungen **12**, **13** und **15** keinen Effekt ausüben.

### Beispiel 20: Inhibierung der Topoisomerase II

Die Substanz **1** wurde in zwei unterschiedlichen in vitro Tests auf Hemmung von Topoisomerase II überprüft.

### • kDNA Assay zur Testung von Topoisomerase II Aktivität :

In diesem von P. Arimondo (Anti-Cancer Drug Design 2000, 15 (6), 413-421) beschriebenen Assay wird kDNA mit humaner DNA-Topoisomerase II in Abwesenheit oder Gegenwart der Testverbindungen behandelt. Die Testung der erfindungsgemäßen Verbindung **1** erfolgte hierbei bei drei unterschiedlichen Konzentrationen (100, 31.6 und 10 *µ*M). Zum Vergleich setzte man eine Positivkontrolle und die Referenzverbindungen m-Amsacrin (m-Amsa), Paclitaxel (Taxol) und Vincristine bei einer jeweiligen Konzentration von 100*µ*M ein.

### Durchführung Assay:

Zu 1*µ*L vorgelegter Testsubstanz (20fach konzentriert in 100% DMSO) werden 2*µ*L 10x Assay Puffer, 1*µ*L kDNA (200ng), 0,5*µ*L human Topoisomerase II (1unit) und 15,5*µ*L H₂O pipettiert und gemischt.

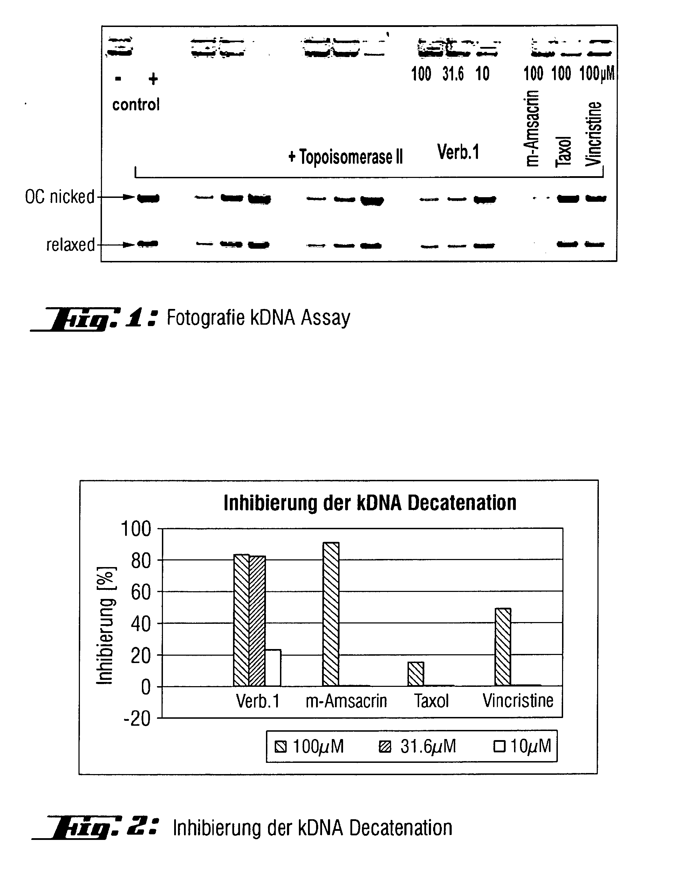

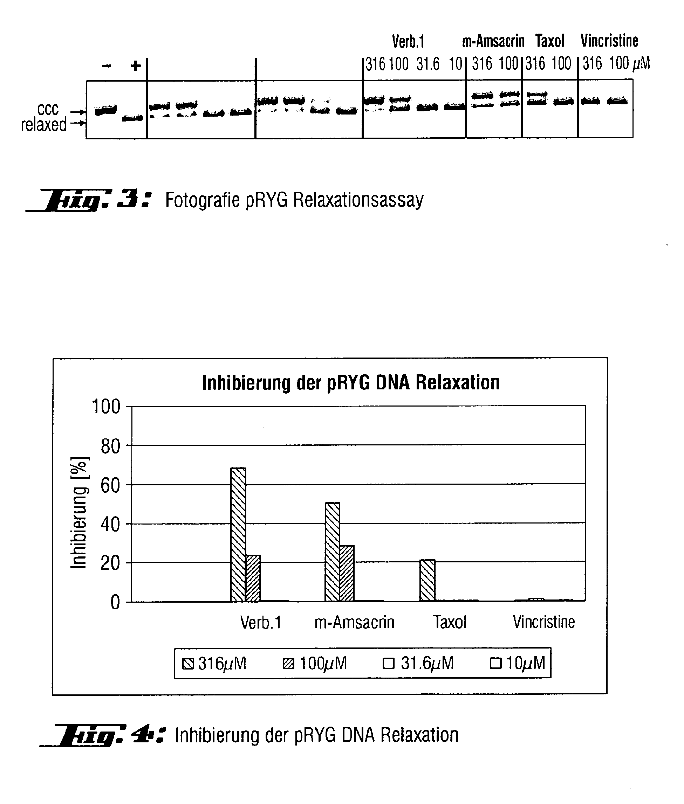

Die Reaktionsansätze werden in den auf 37°C vorgeheizten Heizblock gestellt und 10min bei 37°C inkubiert. Die Inkubation wird nach Zugabe von 4*µ*L 5x Stop Puffer gestoppt und nachfolgend die Substanz mit CIA extrahiert. Danach werden 20*µ*L des Überstandes auf ein 1 % Agarosegel mit 0,25*µ*g/mL Ethidiumbromid aufgetragen und 1 h bei 100V aufgetrennt. Zum Schluss wird das Gel unter UV-Anregung fotografiert (s. Figur 1). Die Quantifizierung der Inhibierung der Decatenation von kDNA erfolgt mit der GelPro®Analyzer Software (s. Figur 2)

### • pRYG Relaxationsassay zur Testung von Topoisomerase II Aktivität:

Mit diesem Relaxationsassay konnte zusätzlich die inhibitorische Eigenschaft der erfindungsgemäßen Verbindungen auf Topoisomerase II gezeigt werden. Die Testung der erfindungsgemäßen Verbindung 1 erfolgte hierbei bei drei unterschiedlichen Konzentrationen (100, 31.6 und 10 *µ*M). Zum Vergleich setzte man die Referenzverbindungen m-Amsacrin, Paclitaxel (Taxol) und Vincristine bei Konzentrationen von 316 und 100*µ*M ein.

Die Durchführung erfolgt hierbei wie folgt:

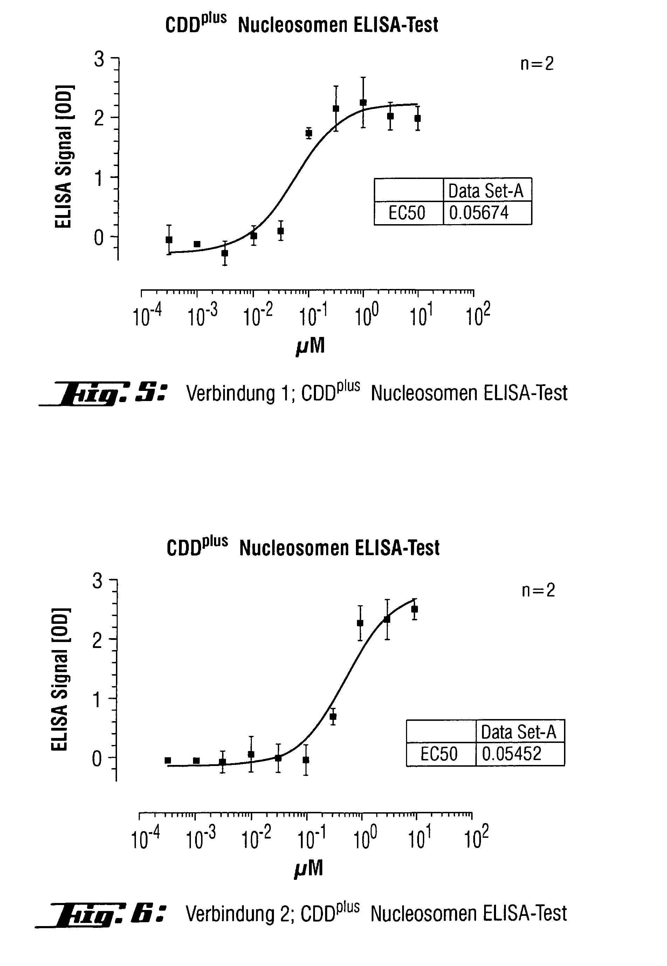

Zu 1*µ*L vorgelegter Testsubstanz (20fach konzentriert in 100% DMSO) werden 2*µ*L 10x Assay Puffer, 0,5*µ*L pRYG DNA (125ng), 0,5*µ*L human Topoisomerase II (1 unit) und 16*µ*L H₂O pipettiert und gemischt. Die Reaktionsansätze werden in den auf 37°C vorgeheizten Heizblock gestellt und 30min bei 37°C inkubiert. Die Inkubation wird nach Zugabe von 4*µ*L 5x Stop Puffer abgestoppt. Danach werden 10*µ*L des Ansatzes auf ein 1,2% Agarosegel mit 0,25*µ*g/mL Ethidiumbromid aufgetragen und 2,5h bei 100V aufgetrennt. Zum Schluß wird das Gel unter UV Anregung fotografiert (s. Figur 3). Die Quantifizierung der Inhibierung der pRYG Relaxation erfolgt mit der GelPro®Analyzer Software (s. Figur 4).

Insgesamt lässt sich festhalten, dass für die erfindungsgemäße Verbindung 1 in den beiden Assays eine signifikante Hemmung der Topoisomerase II nachgewiesen werden konnte. Die Ergebnisse für die Verbindung 1 sind vergleichbar mit den Inhibierungswerten von dem Topoisomerase II-Inhibitor m-Amsacrin. Für Paclitaxel und Vincristine wurden in den beiden Assays erwartungsgemäß keine inhibitorischen Effekte beobachtet.

### Beispiel 21: Zellzvklusanalvse

Der Zellzyklus umfasst die Entwicklung der Zelle von einer Zellgeneration zur nächsten.

Während der Ruhephase (G0) und präsynthetischen Phase (G1) besitzt die Zelle einen diploiden Chromosomensatz (2c). In der Synthesephase (S) wird die DNA-Menge durch Replikation vermehrt. Die S-Phase endet mit Erreichen der prämitotischen Phase (G2M), in der die Zelle einen reduplizierten Chromosomenbestand (4c) und verdoppelten DNA-Gehalt aufweist. In der nachfolgenden, kurzdauernden Mitosephase (M) kommt es zur gleichmäßigen Aufteilung der reduplizierten Chromosomen auf zwei Tochterzellen, die dann jeweils wieder einen diploiden DNA Gehalt zeigen und sich in der G01-Phase befinden, so daß der Zellzyklus neu beginnen kann.

Für die Zellzyklusanalyse wurden KB/HeLa Zellen mit den Testsubstanzen in unterschiedlichen Konzentrationen (0.1-1000 nM) 24 Stunden bei 37°C behandelt.

Der prozentuale Anteil der in der G2/M Phase des Zellzyklus arrettierten Zellen nach Behandlung mit Referenz- oder ausgewählten Testsubstanzen ist in der nachfolgenden Tabelle 4 dargestellt. Die Ergebnisse wurden mit einer speziellen Analysesoftware (ModFit™) ausgewertet.

**Tabelle 4:**

| Hemmkonzentration für einen 50% Anteil der Zellen in der G2/M Phase. | |
|---|---|
| **Beispiel** | **EC50 in nM (50% cells in G2/M)** |
| **1** | **25.2** |
| **2** | 125.3 |
| **4** | 252 |
| **11** | 41.8 |
| **14(V)** | > 1000 |
| **Paclitaxel** | 26.9 |
| **Mitoxantron** | 25.3 |

Die erfindungsgemäßen Verbindungen 1, 2, 4 und 11 weisen vergleichbare Aktivitäten wie die Referenzverbindungen Paclitaxel und Mitoxantron auf.

### Beispiel 22: Nachweis von Apoptose

### CDD^{plus} Nucleosomen ELISA Test:

Die Kernfragmentierung ist eine Spätfolge apoptotischer Prozesse. Die dabei zu beobachtenden Veränderungen lassen sich auf durch Endonukleasen gespaltene DNA-Stränge und daraus resultierende Fragmentierung in Nucleosomen Partikel zurückführen.

Zum Nachweis der Nucleosomen Partikel wurde der von der Firma Roche Molecular Biochemicals beschriebene CDD^{plus} Nucleosomen ELISA Test eingesetzt. Hierzu untersuchte man die Verbindungen **1** und **2** an der U-937 Zelllinie bei unterschiedlichen Konzentrationen (1nM-10*µ*M, 24 h Behandlung). (s. Figur 5 und Figur 6)

Bei den Verbindungen **1** und **2** konnte hierbei ein konzentrationsabhängiger Anstieg von Nucleosomen im Zelllysat beobachtet werden. Im Zellkulturüberstand war kein signifikanter Anstieg nachweisbar, was einen Beleg für einen apoptotischen Zelltod nach Behandlung mit **1** und **2** darstellt.

### Beispiel 23: Nachweis der Sättigungslöslichkeit in Wasser für die erfindungsgemäßen Verbindungen

Die Bestimmung der Sättigungslöslichkeit in Wasser für die Verbindungen **1, 2, 10** und **14** erfolgte wie nachfolgend beschrieben. Zum Anlösen der Substanzen und um die Benetzung der Proben zu verbessern, wurde maximal 1 % DMSO zugesetzt. Zur Überprüfung des Gehaltes wurde eine HPLC-UV-Methode angewandt. Die Ergebnisse sind in der nachfolgenden Tabelle 5 zusammengefasst:

**Tabelle 5:**

| Sättigungslöslichkeiten der Verbindungen **1, 2, 10** und **14** | |
|---|---|
| Verbindungsname | Sättigungslöslichkeit in Wasser [µg/ml] |
| **1** | 25.0 |
| **2** | 28.5 |
| **10 (V)** | 0.038 |
| **14 (V)** | 0.35 |

Die erfindungsgemäßen Verbindungen **1** und **2** zeichnen sich im Vergleich zu den Verbindungen **10** und **14** durch verbesserte Wasserlöslichkeiten aus.

### Beispiel 24: in vivo Aktivität

Die in vivo Aktivität und Verträglichkeit der erfindungsgemäßen Verbindung **2** wurde im Vergleich zu den Substanzen **10** und **14** in einem humanen Xenograft-Modell (Melanom, MEXF462) überprüft. Die Ergebnisse sind in der nachfolgenden Tabelle 6 zusammengefasst:
in vivo Aktivitäten von Verbindung 2, 10 and 14 (Melanom; MEXF462)

**Tabelle 6:**

| In vivo Aktivitäten von Verbindung 2, 10 and 14 (Melanom; MEXF462) | | | | |
|---|---|---|---|---|
| **Verbindung** | **Dosis [mg/kg]** | **Applikation** | **Todesfällen**^{**1**} | **Optimale T/C % (Tag)** |
| **2** | 80 | p.o. | 0/6 Mäuse | 0.0% (18) komplette Remission bei allen 6 Tieren |
| **10** (V) | 70 | p.o. | 5/6 Mäuse tot | 2.3% (7) |
| **10** (V) | 55 | p.o. | 2/6 Mäuse tot | 0.8% (14) |
| **14** (V) | 32 | p.o. | 3/5 Mäuse tot | 14.6% (7) |
| **14** (V) | 16 | p.o. | 3/5 Mäuse tot | 0.7% (18) |

| | | | | |
|---|---|---|---|---|
| 1) Anzahl der verstorbenen Tiere gegenüber der Gesamtzahl | | | | |

Für die Verbindung **2** wurde in diesem Xenograft-Modell eine komplette Remission der Tumore in den behandelten Tieren bei zudem sehr guter Verträglichkeit beobachtet (kein Todesfall). Für die Verbindungen **10** und **14** wurden vergleichbare antitumorale Effekte festgestellt bei jedoch verschlechterten Verträglichkeiten.

### Beispiel 25: in vivo Aktivität

Die in vivo Aktivität und Verträglichkeit der erfindungsgemäßen Verbindung **2** wurde im Vergleich zu der Substanz **10** in einem weiteren humanen Xenograft-Modell (Mamma, MAXF857) überprüft.
Die Ergebnisse werden in der nächsten Tabelle gezeigt:
Effect of 2 and 10 on the Mammary Cancer MAXF857

**Tabelle 7:**

| in vivo Aktivitäten von Verbindung 2 und 10 (Mamma; MAXF857) | | | | |
|---|---|---|---|---|
| **Verbindung** | **Dosis [mg/kg]** | **Applikation** | **Todesfälle n**^{**1**} | **Optimale T/C % (Tag)** |
| 2 | 80 | p.o. | 0/6 Mäuse | 9.6% (10) |
| 10 (V) | 40 | p.o. | 2/6 Mäuse tot | 6.5% (10) |

| | | | | |
|---|---|---|---|---|
| 1) Anzahl der verstorbenen Tiere gegenüber der Gesamtzahl | | | | |

Für die Verbindungen **2** und **10** wurden vergleichbare antitumorale Effekte beobachtet, jedoch ist die Verträglichkeit der Substanz **10** (2/6 Mäusen verstorben) im Vergleich zu **2** wesentlich schlechter.

## Patentansprüche

1. Indol-Derivate der allgemeinen Formel **I** 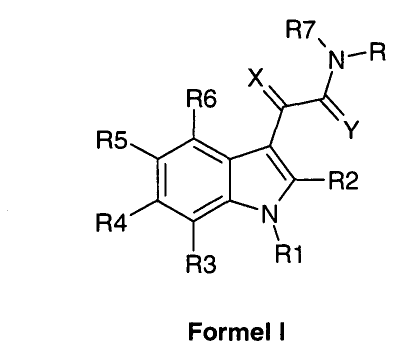 worin
R: für einen oder mehrere Heteroatome ausgewählt aus der Gruppe N, O und S enthaltenden gesättigten, ungesättigten oder aromatischen direkt mit dem Amidstickstoff verbundenen substituierten oder unsubstituierten (C₂-C₁₄)-Heterocyclus steht,
R1: unsubstituiertes oder substituiertes Alkyl-Aryl,
R2:
i) Wasserstoff,
ii) unsubstituiertes oder substituiertes (C₁-C₆)-Alkyl,
R3-R6:
i) Wasserstoff
ii) unsubstituiertes oder substituiertes (C₁-C₆)-Alkyl,
iii) unsubstituiertes oder substituiertes (C₃-C₇)-Cycloalkyl,
iv) Amino, Mono-(C₁-C₄)-Alkylamino, Di-(C₁-C₄)-Alkylamino,
v) Halogen,
vi) mit ein oder mehreren Fluoratomen substituiertes (C₁-C₄)-Alkyl, vorzugsweise Trifluormethylgruppe,
vii) Cyano, geradkettiges oder verzweigtes Cyano-(C₁-C₆)-Alkyl,
viii) (C₁-C₆)-Alkylcarbonyl,
ix) Carboxyl, (C₁-C₄)-Alkoxycarbonyl, Carboxy-(C₁-C₆)-Alkyl oder (C₁-C₆)-Alkoxycarbonyl-(C₁-C₆)-Alkyl,
x) Hydroxy,
xi) -(C₁-C₆)-Alkoxy,
xii) Aryl-(C1-C4)-Alkoxy, vorzugsweise Benzyloxy,
xiii) (C₁-C₆)-Alkoxycarbonylamino, (C₁-C₆)-Alkoxycarbonylamino-(C₁-C₆)-Alkyl,
R7: (C₁-C₆)-Alkylcarbonyl oder (C₁-C₆)-Alkoxycarbonyl
und
X,Y: Sauerstoff oder Schwefel bedeuten,
mit der Maßgabe, wenn R für eine unsubstituierte oder substituierte 2-, 3-, 4-, 5- oder 6-Pyridyl-Gruppe steht und R1-R6 oben genannte Bedeutung hat, steht R7 nicht für einen Acetyl-Rest oder eine *tert*-Butyloxycarbonylgruppe;
deren Tautomere, Stereoisomere, einschließlich der Diastereomere und Enantiomere, sowie die physiologisch verträglichen Salze davon.

2. Indol-Derivate nach Anspruch 1, worin R
i) unsubstituiertes oder substituiertes 5-,6-,7-Chinolyl,
ii) unsubstituiertes oder substituiertes 2-,3-,6-, 7- und 8-Pyridopyrazinyl,
iii) unsubstituiertes oder substituiertes 3-, 4-, 5-, 6- und 7-Indazolyl,
iv) unsubstituiertes oder substituiertes 2-,3-,4-,5- und 6-Pyridyl,
v) unsubstituiertes oder substituiertes 3-, 4- und 5-Isoxazolyl,
vi) unsubstituiertes oder substituiertes 3-, 4- und 5-Isothiazolyl bedeutet,

3. Indol-Derivate gemäß Anspruch 1 oder 2, **dadurch gekennzeichnet, dass** R7 Methoxycarbonyl, Ethoxycarbonyl, Propoxycarbonyl, Acetyl oder Propionyl ist.

4. Indol-Derivate gemäß einem oder mehreren der Ansprüche 1 bis 3, **dadurch gekennzeichnet, dass** die Verbindung der allgemeinen Formel I ausgewählt ist aus der folgenden Gruppe von Verbindungen:
N-{2-[1-(4-Chlor-benzyl)-1H-indol-3-yl]-2-oxo-acetyl}-N-chinolin-6-yl-acetamid **(2)**
{2-[1-(4-Chlor-benzyl)-1*H*-indol-3-yl]-2-oxo-acetyl}-chinolin-6-yl-carbaminsäure- methylester **(3)**
{2-[1-(4-Chlor-benzyl)-1*H*-indol-3-yl]-2-oxo-acetyl}-chinolin-6-yl-carbaminsäure- ethylester **(5)**
{2-[1-(4-Chlor-benzyl)-1*H*-indol-3-yl]-2-oxo-acetyl}-chinolin-6-yl-carbaminsäure- propylester **(6)**
*N*-{2-[1-(4-Chlor-benzyl)-1*H*-indol-3-yl]-2-oxo-acetyl}-*N*-chinolin-6-yl-propionamid **(7)**
{2-[1-(4-Chloro-benzyl)-1*H*-indol-3-yl]-2-oxo-acetyl}-pyridin-4-yl-carbaminsäure-ethylester **(8)**

5. Indol-Derivate der allgemeinen Formel I worin
R: direkt mit dem Amidstickstoff verbundenes
(i) substituiertes 6-Chinolyl, unsubstituiertes oder substituiertes 7-Chinolyl, wobei 2-Methyl-6-Chinolyl ausgeschlossen ist, wobei wenn X ein Schwefelatom ist, R auch nicht substituiertes 6-Chinolyl sein kann.
(ii) unsubstituiertes oder substituiertes 2-,3-,6-, 7- und 8-Pyridopyrazinyl,
(iii) unsubstituiertes oder substituiertes 3-, 4-, 5-, 6- und 7-Indazolyl,
R1: unsubstituiertes oder substituiertes Alkyl-Aryl,
R2: Wasserstoff
R3-R6:
(i) Wasserstoff
(ii) unsubstituiertes oder substituiertes (C₁-C₆)-Alkyl,
(iii) unsubstituiertes oder substituiertes (C₃-C₇)-Cycloalkyl,
(iv) Amino, Mono-(C₁-C₄)-Alkylamino, Di-(C₁-C₄)-Alkylamino,
(v) Halogen,
(vi) mit ein oder mehreren Fluoratomen substituiertes (C₁-C₄)-Alkyl, vorzugsweise Trifluormethylgruppe,
(vii) Cyano, geradkettiges oder verzweigtes Cyano-(C₁-C₆)-Alkyl,
(viii) (C₁-C₆)-Alkylcarbonyl,
(ix) Carboxyl, (C₁-C₄)-Alkoxycarbonyl, Carboxy-(C₁-C₆)-Alkyl oder (C₁-C₆)-Alkoxycarbonyl-(C₁-C₆)-Alkyl,
(x) -(C₁-C₆)-Alkoxy,
(xi) Aryl-(C₁-C₄)-Alkoxy, vorzugsweise Benzyloxy,
(xii) (C₁-C₆)-Alkoxycarbonylamino, (C₁-C₆)-Alkoxycarbonylamino-(C₁-C₆)-Alkyl
und
R7 Wasserstoff
X, Y: Sauerstoff oder Schwefel bedeuten,
deren Tautomere, Stereoisomere, einschließlich der Diastereomere und Enantiomere, sowie die physiologisch verträglichen Salze davon.

6. Indol-Derivate gemäß Anspruch 5 , **dadurch gekennzeichnet, dass** die Verbindung der allgemeinen Formel I ausgewählt ist aus der folgenden Gruppe von Verbindungen:
2-[1-(4-Chlor-benzyl)-1H-indol-3-yl]-N-chinolin-6-yl-2-thioxo-acetamid **(11)**,
2-[1-(4-Chlor-benzyl)-1 H-indol-3-yl]-2-oxo-N-pyrido[2,3-b]pyrazin-7-yl-acetamid **(1)**,
2-[1-(4-Chlor-benzyl)-1H-indol-3-yl]-N-(1H-indazol-5-yl)-2-oxo-acetamid **(4).**

7. Indol-Derivate gemäß einem oder mehreren der Ansprüche 1 bis 6, **dadurch gekennzeichnet, dass** R1 4-Chlorbenzyl, R2-R6 Wasserstoff und X,Y Sauerstoff oder Schwefel bedeuten.

8. Arzneimittel enthaltend mindestens eines der Indol-Derivate gemäß den Ansprüchen 1 bis 7.

9. Arzneimittel nach Anspruch 8 enthaltend das Indol-Derivat in einer mikropartikulären oder nanopartikulären Zusammensetzung.

10. Arzneimittel nach Anspruch 8 oder 9 enthaltend das Indol-Derivat und einen pharmazeutisch verwendbaren Träger und/ oder Verdünnungs- und Hilfsstoff in Form von Tabletten, Dragees, Kapseln, Lösungen zur Infusion oder Ampullen, Suppositorien, Pflaster, inhalativ einsetzbaren Pulverzubereitungen, Suspensionen, Cremes und Salben.

11. Verwendung der Indol-Derivate gemäß den Ansprüchen 1 bis 6 zur Herstellung eines Arzneimittels zur Behandlung von Tumorerkrankungen.

12. Verwendung nach Anspruch 11 zur Behandlung bei Tumorerkrankungen mit Arzneimittelresistenz gegen andere Wirkstoffe.

13. Verwendung nach Anspruch 11 zur Behandlung bei Tumorerkrankungen mit metastasierendem Karcinom.
